# EUROPEAN PATENT APPLICATION

(11) **EP 1 468 652 A1**
(43) Date of publication of application: **20.10.2004**
(21) Application number: 03252435.7
(22) Date of filing: 16.04.2003
(51) Int. Cl.: A61B 17/02, A61B 17/58, A61M 29/00, A61B 17/32

(54) **Apparatus for endoscopic spinal surgery**

(71) Applicant: Tsou, Paul M., Santa Monica, California 90404 (US)
(72) Inventor: Tsou, Paul M., Santa Monica, California 90404 (US)
(74) Representative: Miller, James Lionel Woolverton

(57) **Abstract**

A method of and apparatus for performing percutaneous transformal endoscopic lumbar surgery on a patient, includes the steps of creating an opening in the patient's skin, passing at least one tubular cannula through the opening so as to create a soft tissue tunnel, placing a semi-tubular spreader over the at least one tubular cannula inside the soft tissue tunnel, placing a flat blade spreader into an opening formed by the semi-tubular spreader, dilating the opening by spreading apart blades of the flat blade spreader, inserting bone grafts through the opening and into an intervertebral space of the patient.

## Description

### Background of the Invention

### 1. Field of the Invention

The present invention relates generally to methods and apparatus for percutaneous surgery and, more specifically, to a method and apparatus for performing percutaneous transforaminal lumbar and thoracic disc surgery and interbody fusion,andto endoscopic spinal surgery.

### 2. Description of Prior Art

A substantial segment of the population suffers from spinal pain that is caused by degenerative, herniated or protruded intervertebral discs. Intervertebral discs are members of the spinal column that serve as cushions and mobile linkage elements between the individual vertebrae. The acute herniation of an intervertebral disc can lead to the compression of spinal nerve elements within the spinal canal as well as disc surfaces outside of the spinal canal. The problem can cause severe back pain, leg pain, muscle weakness, and possibly bowel and bladder dysfunction.

The traditional surgical method of spinal nerve element decompression is by the transcanal methods of laminectomy or laminotomy. Optical aids such as microscopes, endoscopes or loupes are often used in these processes. The tissue retractor commonly used in this type of surgery is normally constructed with two blades. More recently, a tubular shaped retractor has been used. Traditionally, this procedure has required two to three days of hospitalization after completion of the surgery.

Chronic back pain due to disc failure, without dominant extremity symptoms may also cause chronic functional impairment.

Prior art solutions have surgically fused adjacent vertebrae together by placing bridging bone material from one vertebra above to one vertebrae below the symptomatic disc(s). The bone fusion surfaces may include the posterior vertebral elements, the vertebral end plates or a combination of the two. Sometimes, metal rods and screws have been used to stabilize the subject spinal fusion segment from the posterior approach.

Because of the tremendously invasive nature of many prior art techniques as compared to the present invention, these prior art techniques caused significant access tissue trauma, even when the skin incision was reduced in length.

Using endoscopic transforaminal techniques, a surgeon can operate through a smaller (roughly 8 - 12 millimeters) opening with endoscopic surgical viewing instruments and miniaturized tools.

The preferred method using endoscopic techniques and miniaturized instrumentation results in an even less intrusive procedure. Because the access surgical trauma and destabilization are less with this technique, endoscopic transforaminal surgery requires a shorter rehabilitation time.

The preferred technique of the present invention adopts an extraspinal canal approach for the correction of spinal conditions, herniated discs and chronic disc pain. In using this approach, the perils of nerve element and dural from sharp trauma and retraction trauma are greatly reduced. The working channel for simple herniated disc extraction is approximately 8mm in diameter and the diameter is somewhat larger for fusion surgery.

Because of the ultra miniaturization of the instruments, the procedure can be performed using local anesthetic agents and conscious sedation. Unlike prior art, overnight hospital stays are not necessary.

In an alternative methodology, the body is opened up as much as necessary. The remaining portion of the access to the target site is conducted using the minimally invasive techniques and tools as described herein. This surgical technique is less invasive than prior art, but more invasive than the preferred methodology of this invention.

To fuse adjacent vertebrae, bone graft material is placed in the evacuated disc space between the bony end-plate of the target vertebrae. After insertion of the structural bone graft material and any additional non-structural osteogenic agents, ingrowth of new autologous bone gradually replaces the graft material to create a unified structure that includes the first and last vertebrae in the fusion segment. Prior art techniques have used structural angular bone blocks, metallic cages, carbon fiber blocks or bone chips that are inserted into the intervertebral space(s). Prior art laparoscopic anterior lumbar fusion technique uses cylindrical metallic cages or bone dowels. These cylindrical shaped devices do not achieve maximum surface contact with the flat surface of the host end plate bed. Thus, seating of cylindrical/round shaped fillers requires end-plate cutting. Surgical end-plate cutting structurally weakens the end-plate and introduces the probability of metallic fillers settling into the soft vertebral cancellous body. In contrast, the preferred mode of the present invention uses modular discoid shaped fillers that do not need end-plate cutting for seating and stability.

However, prior art lateral approached spinal, square shaped graft delivery tubes are bulky. The dimensions of block graft delivery via a prior art square tube can not take full advantage of the maximum outer dimensions of the delivery tube. Additionally, these prior art systems have no method for graft insertions into the L5-S1 disc space. Because prior art minimally invasive systems require generally round tube delivery conduit, the subsequent graft shape is necessarily round/cylindrical as well.

One specific prior art technique, using a rounded filler, is discussed in United States Patent No. 6,217,509 (the '509 patent). The '509 patent describes an access tubular channel from the skin to the targeted work area (which is only used in the posterior transcanal spinal approaches). The working channel inside the tube allows for the use, as needed, of a viewing element, operating tools, tissue retractors, suction channels and a fluid channel. This method is considered more problematic when used in any other approach. According to the '509 patent, a fluid working environment is not desirable in posterior lumbar surgery. However, a fluid environment is utilized in the present invention without degrading endoscopic vision during the ablation of bone, collagenous tissue or bleeder coagulation. A Holmium-YAG laser, used in a fluid medium, in the present invention, eliminates the problems that encountered by the '509 method.

Additionally, the '509 patent does not identify the necessary skin entry location for instruments insertion nor the safe portal into the vertebral annulus. The present invention describes a skin window localization method, identified the safe foraminal annular window and the trajectory for the instruments.

In sequential steps the working cannula, viewing element and all other operating tools enter the same windows. In addition the deep end of the duty cannula is anchored in the opening of the annular window.

Finally, the '509 method neither describes nor allows for the delivery of modular discoid shaped bone graft material (i.e., components of the module are rectangular or have round edges that face the interior of annulus fibrosus).

Therefore, a surgical method, preferred shaped tool(s) and preferred shaped conduits are needed that allow modular discoid shaped filler components, of variable shape and size, to be implanted into an intervertebral disc space using percutaneous endoscopic transforaminal spinal surgery methodology.

### Brief Summary of the Invention

According to the present invention, a method and apparatus are described percutaneous endoscopic transforaminal spinal surgery can use modular discoid shaped components as filler for fusion material, in the intervertebral disc space in the spinal column. In the preferred mode of the invention, the desired final implant composite from the components is discoid in general contour. The individual component(s) have round edges facing the interior of annulus fibrosus and straight edges facing each other. From the frontal view, the components are either half moon or rectangularly shaped.

In the preferred embodiment, the method and apparatus include innovative tools to allow the implantation of the above-mentioned implants into the spinal intervertebral spaces. Also, in the preferred embodiment, the methodology requires the patient to be awake during the procedure. A local anesthetic agent is used to infiltrate the skin window, subcutaneous tissue and trajectory tract with a 6" long, 18-gauge needle and continuing towards the foraminal annular window. The skin window localization is determined by the index disc inclination and the measured length from the center of the disc to the posterior skin surface. The needle insertion trajectory is 25 - 30 degrees in relationship to the frontal plane in line with the disc inclination. After the foraminal annular window placement of the needle, a thin guide wire is inserted through the needle channel and advanced into the center of the disc.

After the guide wire is accurately positioned, the needle is removed and a cannulated obturator is introduced over the proximate tip of the guide wire and inserted toward the annulus at the foramen. The obturator is then advanced through the annulas at the foraminal location. The tapered tip of the obturator should be positioned within the annulus. The guide wire is then removed and a beveled cannula, which allows greater viewing aperture, is inserted over the obturator. Once the beveled tip of the cannula is well within the annulas, the obturator is removed.

If the methodology is utilized to extract herniated spinal disc material, the herniated nucleus pulposus fragments are excised. An operative endoscope is inserted into the cannula and a working tunnel and cavity are created under the herniated elements. The annular collar is opened using mechanical forceps or a Holmium-YAG laser. The herniated fragment is retrieved through the working space and cannula and the epidural and interdiscal spaces are inspected for completeness of removal of the herniated fragments.

When the spinal pathology requires a fusion procedure, the circular shaped annular fenestration is progressively enlarged by inserting progressively larger diameter fish mouth shaped cannula through the skin. In sequential steps, the last smaller cannula is removed after the next larger cannula is inserted. When the largest anatomically feasible foraminal annular window is dilated with the fish mouthed cannula dilation system, the second largest fish mouthed cannula is kept within the annular opening. The largest and the last inserted cannula is removed and replaced by a same sized semi-tubular fish mouthed spreader. The semi-tubular spreader is next inserted with its blades in the completely closed position. The last fish mouthed cannula in the annular opening is then removed.

With the semi-tubular spreader in place, the annular opening is further dilated. Progressively larger diameter solid rods are placed in the channel portion of the semi-tubular spreader until the opening is dilated to the largest anatomically feasible size.

Once the semi-tubular spreader has achieved maximum dilation, excavation of the nucleus pulposus can be performed. Typically, this includes complete removal of the nucleus pulposus and the vertebral cartilaginous end plates to create a natural discoid shaped cavity for the placement of the preferred modular discoid shaped bone graft components.

After the nucleous pulposus has been excavated from the intervertebral disc space, a flat blade spreader is inserted into the channel of the semi-tubular spreader such that it engages the rims of the vertebrae. Both spreaders are rotated, in unison, ninety degrees so that the blades of the flat blade spreader are oriented in a cephalad-caudad direction. After rotating the spreaders, both the flat and the semi-tubular blades remain momentarily intertwined. The flat blade spreader is moderately dilated by inserting progressively thicker rectangular shaped dilator and the spreading actions exerted on the spreader handles. The semi-tubular spreader is then removed.

Additional spreading of the flat blade spreader continues by using thicker rectangular dilators. Ultimately, the rectangular annular opening in height and width should be 8 - 15 mm.

As has been discussed, the method of the invention is implemented through the use of several tools. Included are covers for the flat blade created tissue tunnel. Several attachment mechanisms, cover to flat blade, are entertained. One is a channel fabricated into the outer surface of the paired flat blades, allowing for the attachment of covers for the open sides of the flat blade spreader. Other attachment mechanisms include clasps and screw-on devises. These fixed attachment methods permit the spreader blades and covers to move as one unit. The rectangular channel, now covered on all four sides, allows smooth passage of maximum sized bone graft without graft entanglement in the soft tissues. Because the entire width of the flat blade spreader is oriented cephalad-caudad inside the disc space, there is no wastage spreader height during the insertion of the bone graft.

A first component of the discoid shaped bone graft is inserted and placed as anteriorly as possible. The first bone graft component is followed by a second. For a small intervertebral space, the second graft component may constitute the final implant. For the larger disc spaces, a rectangular shape modular component can be inserted between the anterior and posteriorly positioned implants. After the structural graft components are in place, the remaining voids of the interspace are filled with non-structural shaped osteoinductive agents.

Further features and advantages of the present invention will be appreciated by reviewing the following drawings and detailed description of the invention.

Various aspects of the invention are set out in the independent. Various optional features are set out in the dependent claims. A number of methods in accordance with the invention will now be discussed.

One procedure comprises a method of performing percutaneous transforaminal endoscopic lumbar surgery on a patient, comprising the steps of: creating an opening in said patient's skin; passing at least one tubular cannula through said opening so as to create a soft tissue tunnel; placing a semi-tubular spreader over a said at least one tubular cannula inside said soft tissue tunnel; placing a flat blade spreader into an opening formed by said semi-tubular spreader; dilating said opening by spreading apart blades of said flat blade spreader; inserting bone grafts through said opening and into an intervertebral space of said patient.

The following optional features may be taken together, or independently.

Preferably, said at least one tubular cannula has a first end, and said first end passes through said opening and stops substantially adjacent to said intervertebral space of said patient.

Preferably, the method further comprises, after said step of placing said semi-tubular spreader, the step of removing said at least one cannula.

The method preferably comprises after said step of dilating said opening, the step of rotating said flat blade spreader and said semi-tubular spreader in unison.

The method preferably comprises after said rotating step, the step of removing said semi-tubular spreader.

Preferably, said flat blade spreader is rotated through an angle of approximately ninety degrees.

Preferably, said flat blade spreader is rotated through an angle such that blades of said flat blade spreader are oriented substantially cephalad-caudad.

Preferably when said flat blade spreader is dilated, a cross section of said soft tissue tunnel is changed to be substantially rectangular in shape, and said intervertebral space is increased.

Preferably, prior to said step of inserting bone grafts, the step of debriding nucleus pulposus in said intervertebral space is provided.

Preferably, said bone grafts are substantially rectangular in shape.

Preferably, said bone grafts have the shape of a flat disc.

Another procedure comprises a method of performing percutaneous transformal lumbar surgery in a patient, comprising the steps of: creating a skin window in said patient's skin; defining a foraminal window substantially adjacent to said a discal space of said patient; inserting a needle through said skin window and guiding a tip of said needle to said foraminal annular window; passing an obdurator over a said needle so as to create a soft tissue tunnel in said patient, said soft tissue tunnel beginning at said skin window and terminating at said foraminal window; removing said needle; placing a first cannula over said obdurator, within said soft tissue tunnel; removing said obdurator; placing a plurality of progressively larger cannulae within said soft tissue tunnel so as to dilate said skin window and said soft tissue tunnel, each of said cannulae being removed after said next larger cannula is inserted; inserting a semi-tubular spreader over one of said tubular cannulae, said semi-tubular spreader having a tube portion comprised of an upper arm and lower arm, said semi-tubular spreader being oriented such that an opening between said upper arm and said lower arm is substantially horizontal; dilating said annular fenestration and vertebral separation by using circular rods of progressively increasing diameter inserted within said tube portion of said semi-tubular spreader; debriding nucleus pulposus within said intervertebral space; inserting a flat blade spreader into said semi-tubular spreader, said flat blade spreader having at least two blade portions, said blade portions fitting into said opening between said upper and lower arms of said semi-tubular spreader; rotating said semi-tubular spreader and said flat blade spreader in unison; removing said semi-tubular spreader; spreading said blade portions of said flat blade spreader so as to dilate said soft tissue tunnel and spread apart vertebrae adjacent to said intervertebral space; inserting at least one substantially rectangular graft substantially anteriorly in said intervertebral space; filling lattices of the block graft and void spaces of the interspace with osteoinductive agents.

Preferably, said skin window constitutes an annular fenestration.

Preferably, said semi-tubular spreader and said flat blade spreader are rotated through an angle of approximately ninety degrees.

A number of preferred embodiments will now be described by way of example with reference to the drawings, in which:
Figures 1A to 1C demonstrate three different C-arm (x-ray) views in the lumbar transforaminal endoscopic approach to the disc.
Figure 2 demonstrates the approach needle trajectory to annular window through a specific skin window determined in the preferred methodology in transforaminal endoscopic lumbar surgery.
Figure 3 presents a transforaminal endoscopic excision technique for a paramedian disc herniation.
Figure 4 presents a view of a disc and its adjacent vertebrae.
Figure 5 demonstrates the preferred transforaminal endoscopic method in alleviated nerve compression in the foramen and the lateral recess.
Figure 5a shows the annular floor of the foramen is being removed using forceps.
Figure 5b shows the foraminal bony roof overhang is being ablated using the Holmium-Yag laser.
Figure 6 presents a semi-tubular spreader, with fish mouthed end, and solid rod dilators.
Figure 7 presents a flat blade spreader.
Figure 8 presents tools to excavate the intervertebral disc space.
Figure 9 presents cylindrical tools used to create a working channel from the skin window into the foraminal annular window.
Figures 10 and 11 present bone graft materials used in the preferred embodiment of the present invention.

### Detailed Description of the Invention

According to the present invention, there is disclosed a method and apparatus for performing percutaneous spinal transforaminal endoscopic interbody fusion using modular discoid shaped graft components.

In the following description, for the purposes of explanation, specific devices, component arrangements and construction details are set forth in order to provide a more thorough understanding of the invention. It will be apparent to those skilled in the art, however, that the present invention may be practiced without these specifically enumerated details and that the preferred embodiment can be modified so as to provide other capabilities, such as the capability for the remote control to operate with other devices. In some instances, well-known structures and methods have not been described in detail so as not to obscure the present invention unnecessarily.

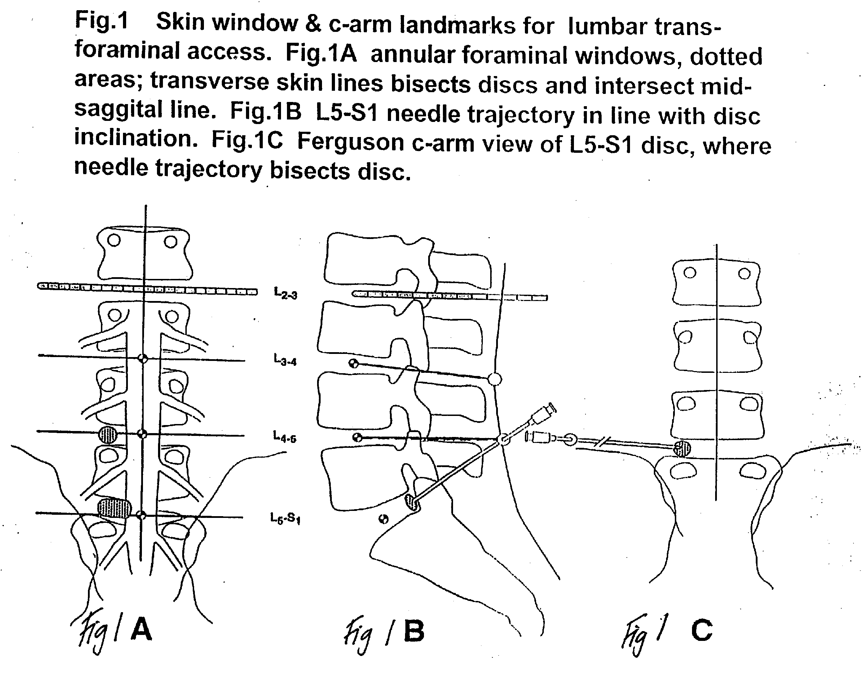

Referring first to Figure 1, an approach to percutaneous transforaminal endoscopic lumbar surgery is demonstrated. As seen in A, B and C, the angle of the approach to the disc spaces varies, depending upon the inclination of the targeted intervertebral disc space. The general method of access utilizes a small diameter cannula to create a soft tissue tunnel from an opening in the skin window to the foraminal annular window or a target area.

In the preferred embodiment of percutaneous transforaminal surgery, the target is the foraminal annular window. As shown in Figure 1, the initial step is to insert a needle following the preferred path from the skin window to the foraminal annular window. The preferred embodiment of the present invention utilizes a large bore needle. It will be apparent to those of skill in the art that the exact size and diameter of the needle can vary and will depend on the particular treatment needs of the patient.

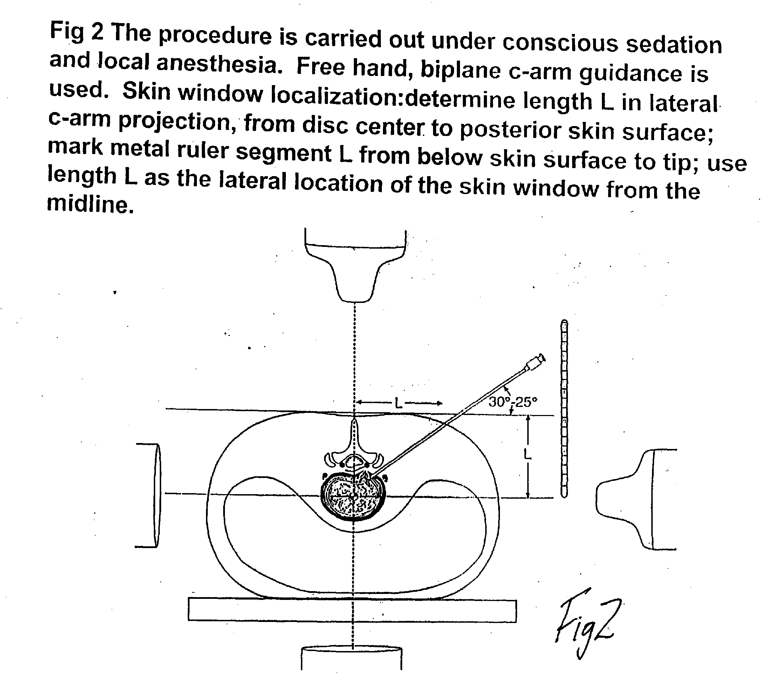

Referring next to Figure 2, in the preferred mode, the invention shows the percutaneous transforaminal endoscopic approach in the axial view. However the invention can also be used for minimally invasive procedures requiring other modified approaches to the spine. The preferred method of localizing the skin window is to plot the L5-S1 disc inclination in the lateral c-arm projection, as demonstrated by the needle position in Figure 1. Referring again to Figure 2, the skin window is determined by measuring distance L from the approximate center of the disc to the posterior skin surface. The distance L is also the distance of skin window from the approximate saggital midline of the patient. The cephalad-caudad location of the skin window is found by the previously plotted disc inclination in the lateral c-arm view.

Initially, a local anesthetic agent is used to infiltrate the skin window, subcutaneous tissue and trajectory tract. As noted above, a needle is then inserted from the skin window at approximately 25-30 degrees from the frontal plane anteromedially toward the foraminal annular window. It has been found that in a typical patient, the an approximately six inch long, eighteen gauge needle provides satisfactory treatment results. The exact size of the needle may, as noted be smaller or larger, depending on the needs of the individual patient.

Although the preferred embodiment of the method of the present invention utilizes an angle of 25-30 degrees from the frontal plane, it will be apparent to those skilled in the art that a larger or smaller angle can be used in instances where the specific anatomy or treatment needs of the patient require. For example, the patient may have a disk herniation in an unusual location which require access from a different orientation. Alternatively, the patient may have a bone structure which precludes inserting the needle at the preferred angle. In such situations, the surgeon will have sufficient knowledge to determine the most advantageous manner and orientation in which to insert the needle.

The needle is advanced toward the target foraminal annular window 12. In the postero-anterior view, the needle tip is placed approximately in the center of the foraminal annular window between the medial and lateral borders of the pedicle in the foramen and then advanced through the full thickness of the annulus.

A guide wire is then inserted through the needle channel.

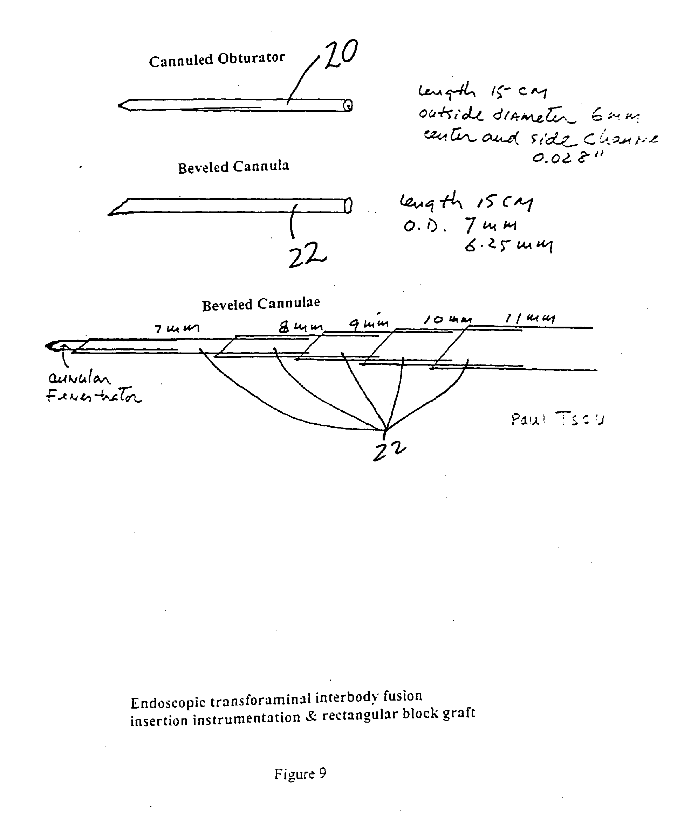

The guide wire is advanced a sufficient distance to be adjacent to the annulus and the needle is removed. In the preferred embodiment, this distance is approximately one centimeter. A bluntly tapered cannulated obturator 20 (see figure 9) is inserted over the guide wire and firmly engages the annulus. The guide wire is then removed.

The thickness of the annulus is infiltrated with local anesthetic agent in four quadrants through the channels of the obturator 20. Next, the through and through fenestration of the annular window is achieved by advancing the bluntly tapered obturator 20. After the taper of the obturator 20 is advanced within the annulus, a beveled cannula 22 is placed over the obturator 20. The cannula 22 is advanced until its beveled tip straddles the annular opening. Then, the obturator 20 is removed.

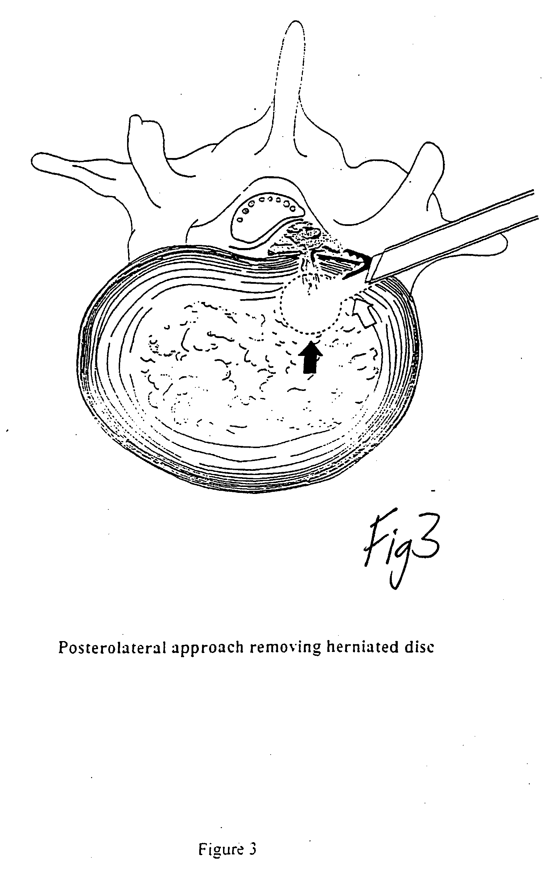

An operating endoscope is inserted into the beveled cannula 22. If the pathology is of intracanal intervertebral herniation, refer to figure 3 for extraction method. Working spaces are created in the annulus. Referring to Figure 3, a working oft tissue tunnel is identified by the open arrow and the solid arrow identifies a working cavity. The biting forceps are positioned to open the herniation annular collar. Once the collar is opened, the surgeon grasps the herniated nucleus fragment and pulls it out via the previously established work spaces.

If the operating pathology calls for fusion, the targeted disc space of the spine is distracted apart further using progressively larger cannulae 22. During the disc space distraction process, the full diameter of the cannulae 22 enters the annular opening. Progressively greater distraction is achieved using sequentially larger cannulae 22. In the preferred embodiment the cannulae 22 are what are commonly known as fish mouth cannulae.

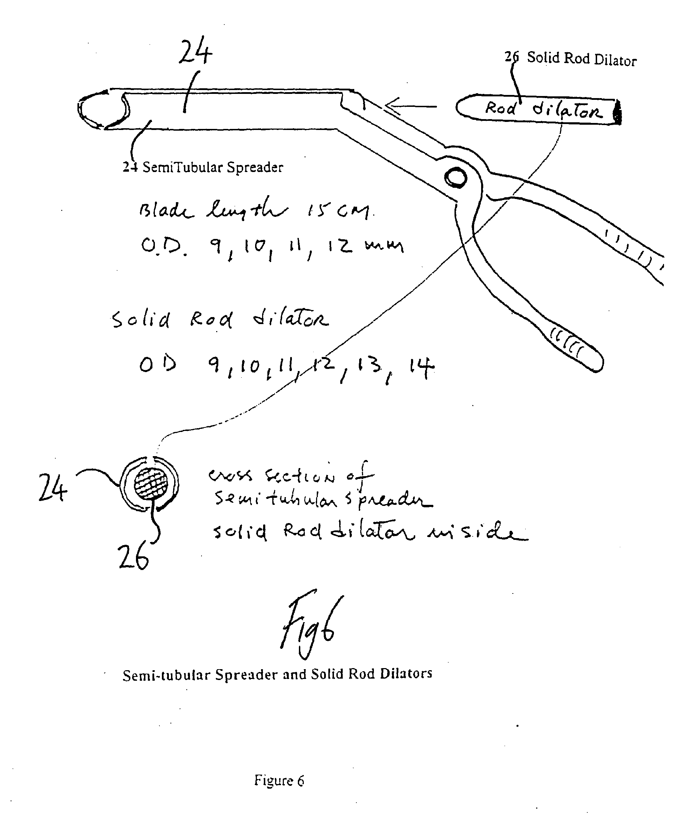

When the disc space height distraction reaches anatomical maximum, a semi tubular spreader 24 (see Figure 6) replaces the largest sized cannula 22. The semi-tubular spreader 24 is opened further using progressively larger solid bore rods 26 until the spreader blades can achieve the desired opening. In the preferred embodiment, the opening is typically in the range of 2.5mm-3.5mm. The exact size necessary will be dependent on the anatomy and treatment needs of the patient. At this point, the rotational orientation of the semi-tubular spreader 24 blades is such that each blade engages the bony rims of the opposing vertebra, and the opening is substantially parallel to the disc. Up to this step, the fenestration made in the annulus remains circular in shape.

Referring next to Figures 3 - 5 and 8, once the semi-tubular spreader 24 is opened in this position, ablation of the nucleus pulposus is initially undertaken using various hand rongeurs, curettes, rake, shaver 30(4) and negative pressure devices. The cartilaginous end-plate and the adjacent nucleus require a more aggressive T-shaped configured debrider 30(2). The T-shaped debriders 30(2), rake and other attachments can be operated by hand or attached to a low speed, high torque power source. The softer central nucleus pulposus removal may be achieved by using a motorized shaver. In the surgical process for an acutely herniated disc, the motorized shaver will debride the posterior nucleus and remove unstable nucleus material from the herniation path. The annular collar may be divided by using a cutting forcep to perform a partial annulectomy in order to access extended nucleus material in the epidural space. The side walls of the annular channel may be further widened and medialized by using a Holmium-Yag laser.

In the methodology of the invention, when the disc removal has reached its desired limits and the selected amount of the nucleus pulposus and cartilagenous end plate have been removed, the excavated cavity will have roughly in the shape of a biconvex and round disc.

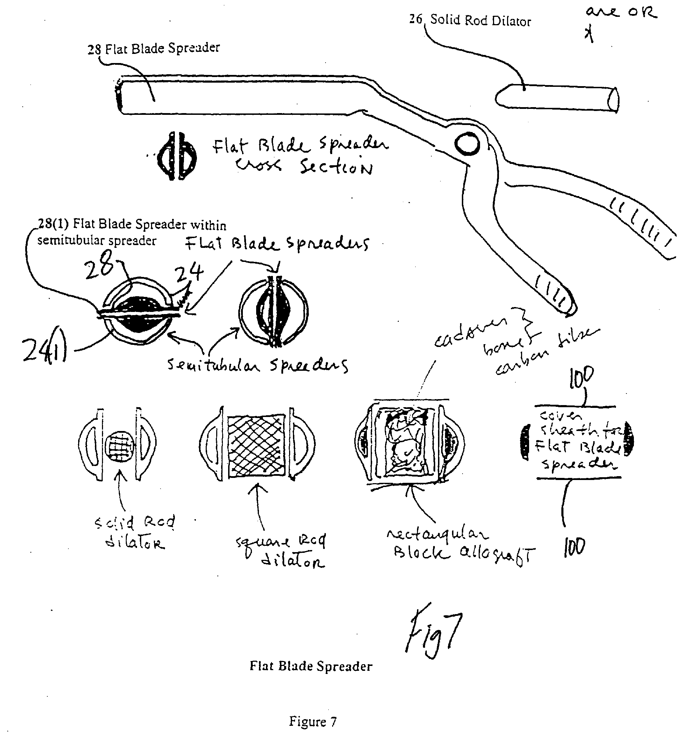

The annular opening thus far is circular in its gross dimensions. Referring next to Figure 7, the shape of the circular annular fenestration is changed, in the subsequent steps, to an angular (i.e., substantially rectangular) opening by the unique methodology of the present invention. In the preferred embodiment, the circular shaped opening is changed to a more angular opening in the shape of a square or rectangle. The angular shaped opening wastes no distracted disc space height dimension and will accept the angular graft components for maximum size and contact surfaces between the graft and the host bed.

Next, a flat blade spreader 28, with blades that are slightly wider than those of the semi-tubular spreader 24, is inserted into the slot of the semi tubular spreader 24(1). The two embraced spreaders 24 and 28 are then rotated through an angle so that the flat blades are oriented substantially cephalad-caudad. In the preferred embodiment of the present invention, the spreaders are rotated through an angle of approximately 90 degrees. While the two spreaders are intertwined, the flat blade spreader 28 is opened moderately and the semi tubular spreader 24 is removed. The flat blade spreader 28 is then opened to its maximum width, using a passive spreading technique. This can be achieved by using progressively thicker rectangular shaped distractor. The opened sides of the tunnel created by the flat blade spreader are covered by attaching cover blades both on the cephadad and the caudad end of the tunnel. The covers blades are incorporated to retract soft tissue and exiting nerve away from the tunnel proper. Several cover attachment mechanisms are entertained. One is that of an attachment tunnel on the outside surface of the flat blade. Other methods of attachment mechanism include clasps and screw-ons. The enclosed tunnel makes transit of the graft components free of entanglement risks in the soft tissue.

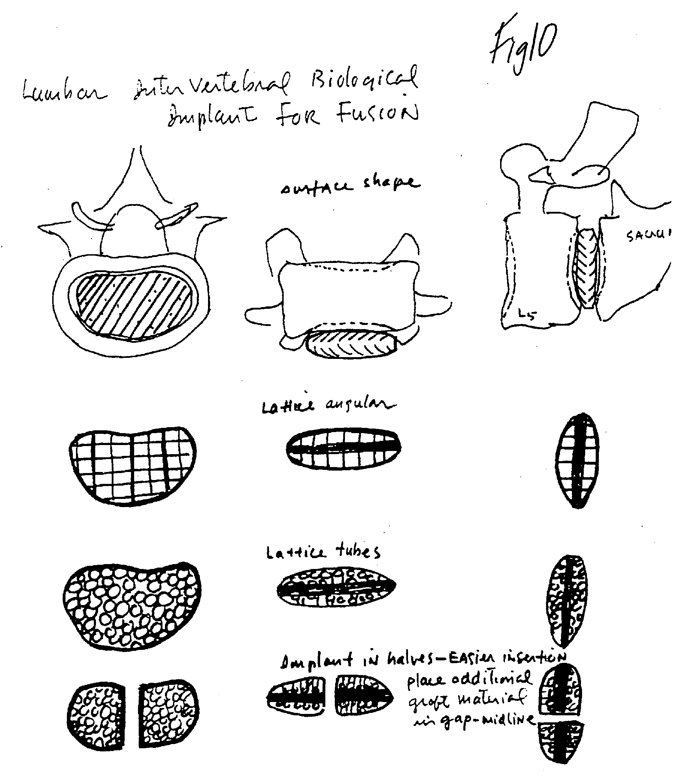

Referring next to the implant/filler material shown in Figure 10, multiple shallow perforations are made in the subchondral bone of both end-plates to allow for the entry of a blood supply for the fusion process.

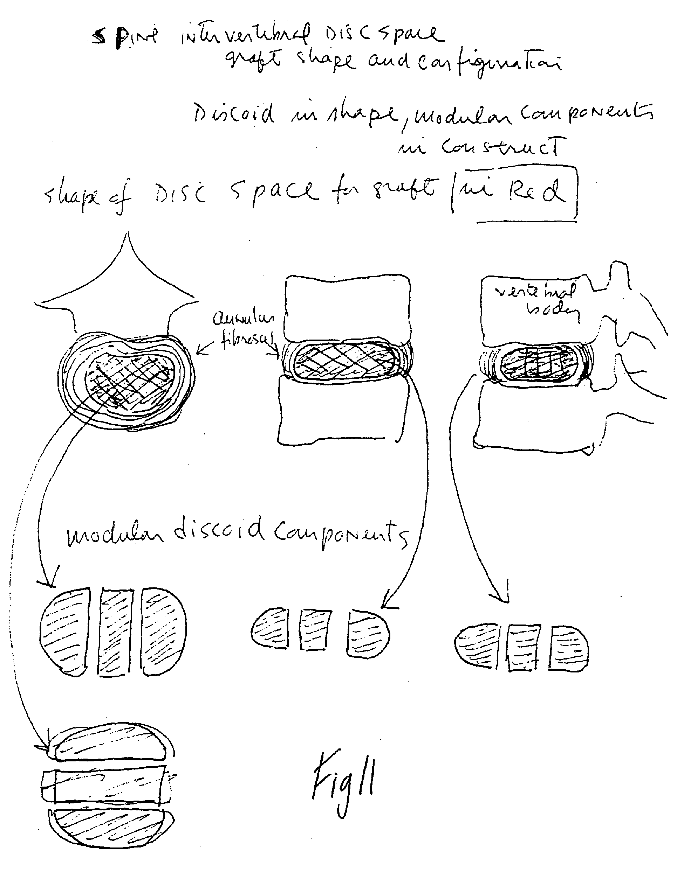

Referring next to Figure 11, after completion of the above preparations, introduction of the optimally sized modular discoid shaped bone graft components from the outside of the skin surface into the disc space is carried out. The preferred method uses at least two pieces of modular discoid graft components. The first piece is inserted and pushed as anteriorly as possible against the interior of the anterior annulus. Gradual seating of the graft is made possible by using various contoured impactors. Once the first graft clears the tips of the flat blade spreader, the second graft is inserted. For disc space of large size, a third graft component, rectangular in shape can be introduced between the first two graft components.

The graft to be inserted should have the largest possible surface area and height to take advantage of the maximum possible contact area with the opposing host end-plates. The bone graft material should be tall enough so that the graft end-plate surfaces are under compression. The ideal vertebral interbody graft shape is that of a disc. Since the tunnel from the skin into the disc space is very limited in height and width, it is preferred to modularize the whole discoid shape into two or more components to facilitate the passage of the material through the relatively smaller tunnel.

After the insertion of the graft material, osteoconductive and osteoinductive supplementary agents in the form of paste, jelly or sponge can also be inserted to fill any small crevices or voids that remain in the target intervertebral disc space.

It will be apparent to those skilled in the art that the foregoing description is for illustrative purposes only, and that various changes and modifications can be made to the present invention without departing from the overall spirit and scope of the present invention. The full extent of the present invention is defined and limited only by the following claims.

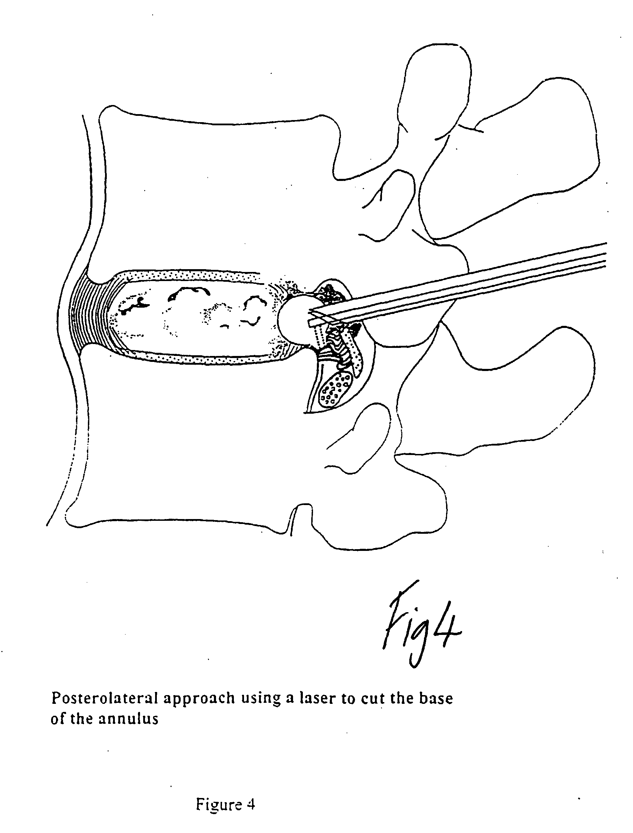

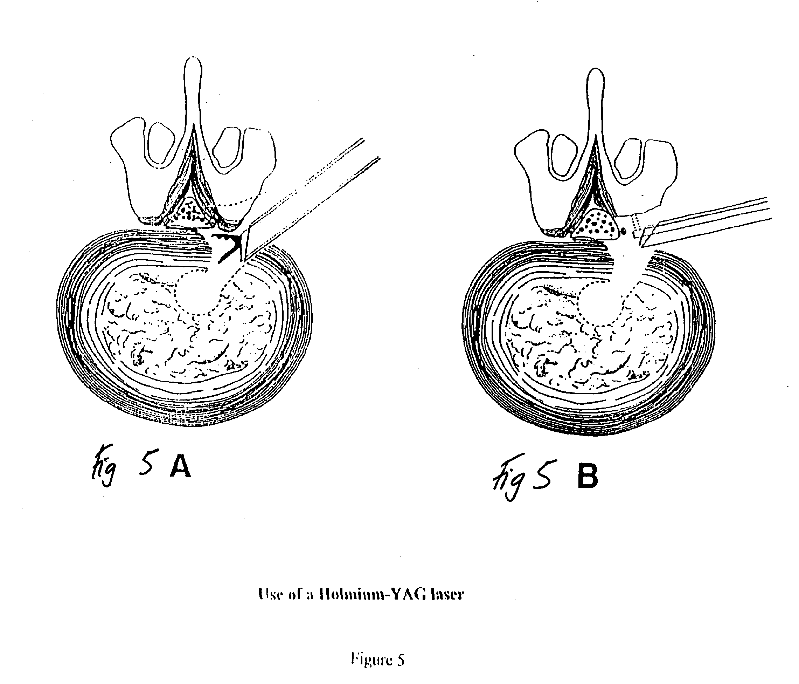

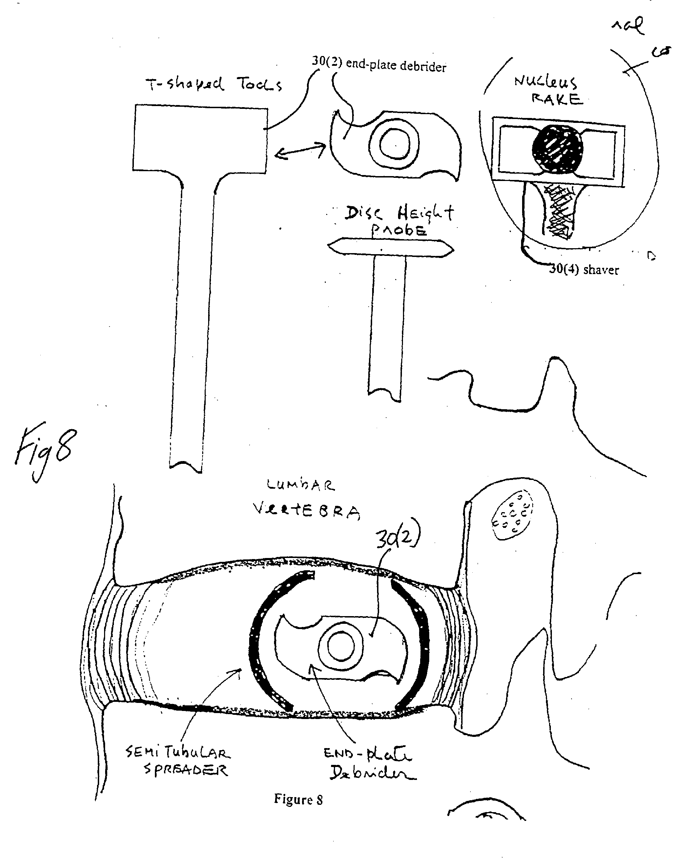

Figure 1 shows skin window and c-arm landmarks for lumbar transforaminal access. Figure 1A shows annular foraminal windows, dotted areas. Transverse skin lines bisect discs and intersect mid-saggital line. Figure 1B shows an L5-S1 needle trajectory in line with disc inclination. Figure 1C shows Ferugson c-arm view of L5-S1 disc, where needle trajectory bisects disc. The procedure of Figure 2 is in the specific embodiment carried out under conscious sedation and local anaesthesia. Free hand, biplane c-arm guidance is used. For skin window localisation, length L may be determined in lateral c-arm projection from disc centre to posterior skin surface. Metal rule may then be marked segment L from below skin surface to tip. Length L may be used as the lateral location of the skin window from the midline. Figure 3 shows a posterolateral approach removing herniated disc. Figure 4 shows a posterolateral approach using a laser to cut the base of the annulus. Figure 5B shows use of a Holmium-YAG laser. In Figure 6, the blade length for the semi-tubular spreader may be 15cm. The semi-tubular spreader may have an outer diameter of 9, 10, 11 or 12mm, for example. The solid rod dilator may have an outer diameter of 9, 10, 11, 12, 13 or 14mm, for example. Thus, it would be appreciated that Figure 6 is a view of semi-tubular spreader and solid rod dilators. The flat blade spreader may have a cover sheet 100 as shown in Figure 7. Figure 8 shows tools for posterolateral endoscopic nucleus pulposis and a vertebral end plate debrider. Figure 9 shows an endoscopic transforaminal interbody fusion insertion instrumentation. In one example, the obturator 20 has a length of 15cm, an outside diameter of 6mm. The bevelled cannula to fit around the obturator may have a length of 15cm, outer diameter of 7mm. Increasingly large cannulae may have outer diameters of 8, 9, 10 and 11mm. As shown in Figure 10, the lumbar intervertebral biological implant for fusion may have an angular lattice, lattice tubes, and/or may be implanted in halves for easier insertion. Additional graft material may be placed in a midline gap. As shown in Figure 11, for a spine intervertebral disc space graft shape and configuration, this may be discoid in shape and modular discoid components may be used to construct the shape of the disc space.

## Claims

1. A flat blade spreader for use in endoscopic lumbar surgery, comprising: a first handle and a second handle joined together by at least one pivot point, each of said handles including a grip; each handle having attached to it a flat surface blade extending outward at an angle from a planar surface of the first and second handle grips.

2. The device of claim 1 wherein said flat surface blades are grooved.

3. An apparatus for debriding nucleus pulposus comprising: a hollow body containing a motor shaft having a distal and a proximate end with said motor shaft being freely rotatable within said body; a rotatable bladed disc coupled perpendicularly to said distal end of said motor shaft; said disc being generally circular in shape and having a plurality of teeth extending outward from said disc; said teeth having a cutting edge on the leading edge of said teeth; and a motor coupled to said proximate end of said motor shaft, which, during operation, causes said bladed disc to rotate.

4. An apparatus for debriding nucleus pulposus comprising: a hollow body containing a motor shaft having a distal and a proximate end with said motor shaft being freely rotatable within said body; a rotatable double bladed rectangle coupled, in the same plane as said shaft, to said distal end of said motor shaft; said disc being generally circular in shape and having a plurality of teeth extending outward from said disc; said teeth having a cutting edge on the leading edge of said teeth; and a motor coupled to said proximate end of said motor shaft, which, during operation, causes said bladed disc to rotate.

5. Apparatus for performing percutaneous transforaminal endoscopic lumbar surgery on a patient, comprising: means for creating an opening in said patient's skin; at least one tubular cannula adapted to be passed through said opening so as to create a soft tissue tunnel; a semi-tubular spreader adapted to be placed over a said at least one tubular cannula inside said soft tissue tunnel; and a flat blade spreader adapted to be placed into an opening formed by said semi-tubular spreader; the flat blade spreader having blades adapted to be spaced apart for dilating said opening.

6. Apparatus as claimed in claim 5 which includes means for inserting bone grafts through said opening and into an intervertebral space of said patient.

7. Apparatus as claimed in claim 5 or claim 6, wherein said at least one tubular cannula has a first end, and said first end is adapted to pass through said opening and stop substantially adjacent to said invertervebral space of said patient.

8. Apparatus as claimed in any one of claims 3 to 7 in which said at least one cannula is adapted to be removed from said opening once said semi-tubular spreader is placed inside said soft tissue tunnel.

9. Apparatus as claimed in any one of claims 5 to 8 in which said flat blade spreader and said semi-tubular spreader are adapted for rotation in unison.

10. Apparatus as claimed in claim 9 in which said semi-tubular spreader is adapted to be removable from said opening after rotation of said spreaders in unison.

11. Apparatus as claimed in claim 9 or claim 10, wherein said flat blade spreader is adapted for rotation through an angle of approximately ninety degrees.

12. Apparatus as claimed in claim 9 or claim 10 or claim 11, wherein said flat blade spreader is adapted for rotation through an angle such that blades of said flat blade spreader are oriented substantially cephalad-caudad.

13. Apparatus as claimed in any one of claims 5 to 12, wherein said flat blade spreader is adapted, upon dilation to change a cross section of said soft tissue tunnel to be substantially rectangular in shape, and to increase said intervertebral space.

14. Apparatus of any one of claims 5 to 13 including means for debriding nucleus pulposus in said intervertebral space prior to bone graft insertion.

15. Apparatus as claimed in claim 6 or any preceding claim when dependent thereon wherein said bone grafts are substantially rectangular in shape.

16. Apparatus as claimed in claim 6 or any preceding claim when dependent thereon, wherein said bone grafts have the shape of a flat disc.

17. Apparatus for performing percutaneous transformal lumbar surgery in a patient, comprising: means for creating a skin window in said patient's skin; means for defining a foraminal window substantially adjacent to said a discal space of said patient; a needle adapted to be inserted through said skin window with a tip of said needle being adapted to be guided to said foraminal annular window; an obdurator adapted to be passed over a said needle so as to create a soft tissue tunnel in said patient, said soft tissue tunnel beginning at said skin window and terminating at said foraminal window; said needle also being adapted for removal from said skin window; a first cannula adapted to be placed over said obdurator, within said soft tissue tunnel; said obdurator also being adapted for removal from said patient; a plurality of progressively larger cannulae adapted to be placed in order of increasing size within said soft tissue tunnel so as to dilate said skin window and said soft tissue tunnel, with each of said cannulae being removable after said next larger cannula is inserted; a semi-tubular spreader adapted to be inserted over one of said tubular cannulae, said semi-tubular spreader having a tube portion comprised of an upper arm and lower arm, said semi-tubular spreader being oriented such that an opening between said upper arm and said lower arm is substantially horizontal; circular rods of progressively increasing diameter adapted for dilating annular fenestration and vertebral separation and for insertion within said tube portion of said semi-tubular spreader; means for debriding nucleus pulposus within said intervertebral space; a flat blade spreader adapted to be inserted into said semi-tubular spreader, said flat blade spreader having at least two blade portions, said blade portions adapted for fitting into said opening between said upper and lower arms of said semi-tubular spreader; said semi-tubular spreader and said flat blade spreader being adapted for rotation in unison; said semi-tubular spreader being adapted for removal from said patient; said blade portions of said flat blade spreader being adapted to be spread so as to dilate said soft tissue tunnel and spread apart vertebrae adjacent to said intervertebral space.

18. Apparatus as claimed in claim 17 including at least one substantially rectangular graft adapted to be inserted substantially anteriorly in said intervertebral space; and means for filling lattices of the block graft and void spaces of the interspace with osteoinductive agents.

19. Apparatus of claim 17 or claim 18, wherein said skin window constitutes an annular fenestration.

20. Apparatus of claim 17 or claim 18 or claim 19, wherein said semi-tubular spreader and said flat blade spreader are rotated through an angle of approximately ninety degrees.

21. A method of performing percutaneous transforaminal endoscopic lumbar surgery on a patient, comprising the steps of: creating an opening in said patient's skin; passing at least one tubular cannula through said opening so as to create a soft tissue tunnel; placing a semi-tubular spreader over a said at least one tubular cannula inside said soft tissue tunnel; placing a flat blade spreader into an opening formed by said semi-tubular spreader; dilating said opening by spreading apart blades of said flat blade spreader; inserting bone grafts through said opening and into an intervertebral space of said patient.

22. A method of performing percutaneous transformal lumbar surgery in a patient, comprising the steps of: creating a skin window in said patient's skin; defining a foraminal window substantially adjacent to said a discal space of said patient; inserting a needle through said skin window and guiding a tip of said needle to said foraminal annular window; passing an obdurator over a said needle so as to create a soft tissue tunnel in said patient, said soft tissue tunnel beginning at said skin window and terminating at said foraminal window; removing said needle; placing a first cannula over said obdurator, within said soft tissue tunnel; removing said obdurator; placing a plurality of progressively larger cannulae within said soft tissue tunnel so as to dilate said skin window and said soft tissue tunnel, each of said cannulae being removed after said next larger cannula is inserted; inserting a semi-tubular spreader over one of said tubular cannulae, said semi-tubular spreader having a tube portion comprised of an upper arm and lower arm, said semi-tubular spreader being oriented such that an opening between said upper arm and said lower arm is substantially horizontal; dilating said annular fenestration and vertebral separation by using circular rods of progressively increasing diameter inserted within said tube portion of said semi-tubular spreader; debriding nucleus pulposus within said intervertebral space; inserting a flat blade spreader into said semi-tubular spreader, said flat blade spreader having at least two blade portions, said blade portions fitting into said opening between said upper and lower arms of said semi-tubular spreader; rotating said semi-tubular spreader and said flat blade spreader in unison; removing said semi-tubular spreader; spreading said blade portions of said flat blade spreader so as to dilate said soft tissue tunnel and spread apart vertebrae adjacent to said intervertebral space; inserting at least one substantially rectangular graft substantially anteriorly in said intervertebral space; filling lattices of the block graft and void spaces of the interspace with osteoinductive agents.
